(19) Europäisches Patentamt
European Patent Office
Office européen des brevets

(11) Veröffentlichungsnummer : **0 421 928 B1**

(12) # EUROPÄISCHE PATENTSCHRIFT

(45) Veröffentlichungstag der Patentschrift : **15.06.94 Patentblatt 94/24**

(51) Int. Cl.$^5$ : **C07C 69/732,** C08K 5/10

(21) Anmeldenummer : **90810736.0**

(22) Anmeldetag : **25.09.90**

(54) **Hydroxyphenylcarbonsäureester als Stabilisatoren.**

(30) Priorität : **02.10.89 CH 3583/89**

(43) Veröffentlichungstag der Anmeldung : **10.04.91 Patentblatt 91/15**

(45) Bekanntmachung des Hinweises auf die Patenterteilung : **15.06.94 Patentblatt 94/24**

(84) Benannte Vertragsstaaten : **BE DE FR GB IT NL SE**

(56) Entgegenhaltungen :
**EP-A- 0 148 729**
**EP-A- 0 333 468**

(73) Patentinhaber : **CIBA-GEIGY AG**
**Klybeckstrasse 141**
**CH-4002 Basel (CH)**

(72) Erfinder : **Pitteloud, Rita, Dr.**
**Sur le Village**
**CH-1724 Praroman (CH)**

EP 0 421 928 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die vorliegende Erfindung betrifft neue Hydroxyphenylcarbonsäureester, die mit diesen Verbindungen gegen thermischen, oxidativen und aktinischen Abbau stabilisierten organischen Materialien sowie die Verwendung der Hydroxyphenylcarbonsäureester als Stabilisatoren.

Dialkyl-hydroxyphenylcarbonsäureester von Di- und Tripentaerythrit und deren Verwendung als Stabilisatoren werden zum Beispiel in US-A-3,642,868 beschrieben. Ferner werden Dialkyl-hydroxyphenylcarbonsäureester in EP-A-366,040 und Derwent-Referat 87-105824/15 offenbart.

Ein Gegenstand der vorliegenden Erfindung sind Verbindungen der Formeln Ia und/oder Ib,

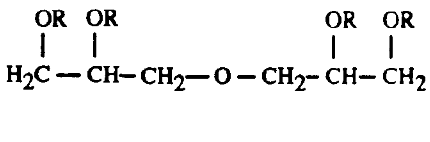 , 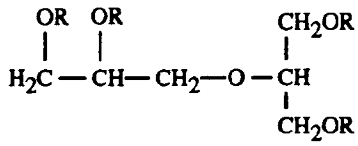

(Ia) (Ib)

worin die Reste R gleich oder verschieden sind und eine Gruppe der Formel II bedeuten,

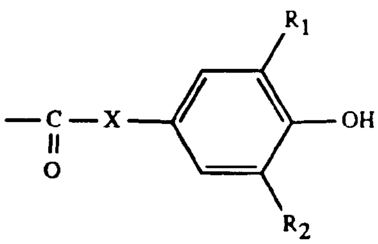 (II)

worin X eine direkte Bindung, Methylen, Ethylen oder eine Gruppe

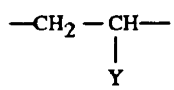

bedeutet und Y $C_1$-$C_8$-Alkyl ist,
$R_1$ und $R_2$ unabhängig voneinander $C_1$-$C_{18}$-Alkyl, $C_5$-$C_{12}$-Cycloalkyl, durch $C_1$-$C_4$-Alkyl substituiertes $C_5$-$C_7$-Cycloalkyl, Phenyl oder $C_7$-$C_{10}$-Phenylalkyl sind und $R_2$ zusätzlich Wasserstoff bedeutet.

Y bedeutet als $C_1$-$C_8$-Alkyl z.B. Methyl, Ethyl, Propyl, Butyl, Pentyl, Hexyl, Heptyl oder Octyl. Geradkettiges Alkyl, insbesondere Methyl ist bevorzugt.

$R_1$ und $R_2$ bedeuten als $C_1$-$C_{18}$-Alkyl z.B. Methyl, Ethyl, Propyl, Isopropyl, Butyl, Isobutyl, tert-Butyl, Pentyl, Hexyl, Heptyl, Octyl, Nonyl, Decyl, Undecyl, Dodecyl, Tridecyl, Tetradecyl, Pentadecyl, Hexadecyl, Heptadecyl oder Octadecyl. $C_1$-$C_4$-Alkyl, insbesondere Methyl und tert-Butyl sind bevorzugt. Besonders bevorzugt sind $R_1$ und $R_2$ tert-Butyl.

$R_1$ und $R_2$ bedeuten als $C_5$-$C_{12}$-Cycloalkyl z.B. Cyclopentyl, Cyclohexyl, Cycloheptyl, Cyclooctyl, Cyclononyl, Cyclodecyl, Cycloundecyl oder Cyclododecyl. $C_5$-$C_7$-Cycloalkyl, insbesondere Cyclohexyl ist eine der bevorzugten Bedeutungen für $R_1$ und $R_2$.

$R_1$ und $R_2$ bedeuten als $C_5$-$C_7$-Cycloalkyl, welches durch $C_1$-$C_4$-Alkyl, bevorzugt Methyl substituiert ist, z.B. Methylcyclohexyl.

$R_1$ und $R_2$ bedeuten als $C_7$-$C_{10}$-Phenylalkyl z.B. Benzyl oder Phenylethyl.

Verbindungen der Formeln Ia und Ib, worin die Reste R gleich sind, sind bevorzugt.

X bedeutet bevorzugt eine direkte Bindung, Methylen oder Ethylen, insbesondere Ethylen.

Von Interesse sind Verbindungen der Formeln Ia und Ib, worin $R_1$ und $R_2$ unabhängig voneinander $C_1$-$C_{12}$-Alkyl, $C_5$-$C_7$-Cycloalkyl, durch $C_1$-$C_4$-Alkyl substituiertes $C_5$-$C_7$-Cycloalkyl, Phenyl, Benzyl oder Phenylethyl bedeuten und $R_2$ zusätzlich Wasserstoff ist.

Ebenfalls von Interesse sind Verbindungen der Formeln Ia und Ib, worin X eine direkte Bindung, Methylen oder Ethylen ist, $R_1$ und $R_2$ unabhängig voneinander $C_1$-$C_4$-Alkyl, Cyclohexyl, durch $C_1$-$C_4$-Alkyl substituiertes Cyclohexyl, Phenyl oder Benzyl bedeuten und $R_2$ zusätzlich Wasserstoff ist.

Verbindungen der Formeln Ia und Ib, worin X Ethylen bedeutet, $R_1$ und $R_2$ unabhängig voneinander $C_1$-$C_4$-Alkyl sind und $R_2$ zusätzlich Wasserstoff bedeutet, sind besonders bevorzugt.

Beispiele für Verbindungen der Formeln Ia und Ib sind:

1,2,6,7-Tetrakis[(3′,5′-di-tert-butyl-4′-hydroxyphenyl)propionyloxy]-4-oxaheptan, 1,5,6-Tris[(3′,5′-di-tert-butyl-4′-hydroxyphenyl)propionyloxy]-2-[(3′,5′-di-tert-butyl-4′-hydroxyphenyl)propionyloxymethyl]-3-oxahexan,
1,2,6,7-Tetrakis[(3′-methyl-5′-tert-butyl-4′-hydroxyphenyl)propionyloxy]-4-oxaheptan, 1,5,6-Tris[(3′-methyl-5′-tert-butyl-4′-hydroxyphenyl)propionyloxy]-2-[(3′-methyl-5′-tert-butyl-4′-hydroxyphenyl)propionyloxymethyl]-3-oxahexan.

Die Verbindungen der Formeln Ia und Ib eignen sich zum Stabilisieren von organischen Materialien gegen thermischen, oxidativen und aktinischen Abbau.

Beispiele für derartige Materialien sind:

1. Polymere von Mono- und Diolefinen, beispielsweise Polypropylen, Polyisobutylen, Polybuten-1, Polymethylpenten-1, Polyisopren oder Polybutadien sowie Polymerisate von Cycloolefinen wie z.B. von Cyclopenten oder Norbornen; ferner Polyethylen (das gegebenenfalls vernetzt sein kann), z.B. Polyethylen hoher Dichte (HDPE), Polyethylen niederer Dichte (LDPE), lineares Polyethylen niederer Dichte (LLDPE).
2. Mischungen der unter 1) genannten Polymeren, z.B. Mischungen von Polypropylen mit Polyisobutylen, Polypropylen mit Polyethylen (z.B. PP/HDPE, PP/LDPE) und Mischungen verschiedener Polyethylentypen (z.B. LDPE/HDPE).
3. Copolymere von Mono- und Diolefinen untereinander oder mit anderen Vinylmonomeren, wie z.B. Ethylen-Propylen-Copolymere, lineares Polyethylen niederer Dichte (LLDPE) und Mischungen desselben mit Polyethylen niederer Dichte (LDPE), Propylen-Buten-1-Copolymere, Propylen-Isobutylen-Copolymere, Ethylen-Buten-1-Copolymere, Ethylen-Hexen-Copolymere, Ethylen-Methylpenten-Copolymere, Ethylen-Hepten-Copolymere, Ethylen-Octen-Copolymere, Propylen-Butadien-Copolymere, Isobutylen-Isopren-Copolymere, Ethylen-Alkylacrylat-Copolymere, Ethylen-Alkylmethacrylat-Copolymere, Ethylen-Vinylacetat-Copolymere oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen; ferner Mischungen solcher Copolymere untereinander und mit unter 1) genannten Polymeren, z.B. Polypropylen/Ethylen-Propylen-Copolymere, LDPE/Ethylen-Vinylacetat-Copolymere, LDPE/Ethylen-Acrylsäure-Copolymere, LLDPE/Ethylen-Vinylacetat-Copolymere und LLDPE/Ethylen-Acrylsäure-Copolymere.
3a. Statistische oder alternierende Copolymere aus $\alpha$-Olefinen und Kohlenmonoxid.
3b. Kohlenwasserstoffharze (z.B. $C_5$-$C_9$) inklusive hydrierte Modifikationen davon (z.B. Klebrigmacherharze).
4. Polystyrol, Poly-(p-methylstyrol), Poly-($\alpha$-methylstyrol).
5. Copolymere von Styrol oder $\alpha$-Methylstyrol mit Dienen oder Acrylderivaten, wie z.B. Styrol-Butadien, Styrol-Acrylnitril, Styrol-Alkylmethacrylat, Styrol-Butadien-Alkylacrylat, Styrol-Maleinsäureanhydrid, Styrol-Acrylnitril-Methylacrylat; Mischungen von hoher Schlagzähigkeit aus Styrol-Copolymeren und einem anderen Polymer, wie z.B. einem Polyacrylat, einem Dien-Polymeren oder einem Ethylen-Propylen-Dien-Terpolymeren; sowie Block-Copolymere des Styrols, wie z.B. Styrol-Butadien-Styrol, Styrol-Isopren-Styrol, Styrol-Ethylen/Butylen-Styrol oder Styrol-Ethylen/Propylen-Styrol.
6. Pfropfcopolymere von Styrol oder $\alpha$-Methylstyrol, wie z.B. Styrol auf Polybutadien, Styrol auf Polybutadien-Styrol- oder Polybutadien-Acrylnitril-Copolymere, Styrol und Acrylnitril (bzw. Methacrylnitril) auf Polybutadien; Styrol, Acrylnitril und Methylmethacrylat auf Polybutadien; Styrol und Maleinsäureanhydrid auf Polybutadien; Styrol, Acrylnitril und Maleinsäureanhydrid oder Maleinsäureimid auf Polybutadien; Styrol und Maleinsäureimid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 5) genannten Copolymeren, wie sie z.B. als sogenannte ABS-, MBS-, ASA- oder AES-Polymere bekannt sind.
7. Halogenhaltige Polymere, wie z.B. Polychloropren, Chlorkautschuk, chloriertes oder chlorsulfoniertes Polyethylen, Copolymere von Ethylen und chloriertem Ethylen, Epichlorhydrinhomo- und -copolymere, insbesondere Polymere aus halogenhaltigen Vinylverbindungen, wie z.B. Polyvinylchlorid, Polyvinylidenchlorid, Polyvinylfluorid, Polyvinylidenfluorid; sowie deren Copolymere, wie Vinylchlorid-Vinylidenchlorid, Vinylchlorid-Vinylacetat oder Vinylidenchlorid-Vinylacetat.

8. Polymere, die sich von a,b-ungesättigten Säuren und deren Derivaten ableiten, wie Polyacrylate und Polymethacrylate, Polyacrylamide und Polyacrylnitrile.
9. Copolymere der unter 8) genannten Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril-Alkylmethacrylat-Butadien-Terpolymere.
10. Polymere, die sich von ungesättigten Alkoholen und Aminen bzw. deren Acylderivaten oder Acetalen ableiten, wie Polyvinylalkohol, Polyvinylacetat, -stearat, -benzoat, -maleat, Polyvinylbutyral, Polyallylphthalat, Polyallylmelamin; sowie deren Copolymere mit in Punkt 1 genannten Olefinen.
11. Homo- und Copolymere von cyclischen Ethern, wie Polyalkylenglykole, Polyethylenoxyd, Polypropylenoxyd oder deren Copolymere mit Bisglycidylethern.
12. Polyacetale, wie Polyoxymethylen, sowie solche Polyoxymethylene, die Comonomere, wie z.B. Ethylenoxid, enthalten; Polyacetale, die mit termoplastischen Polyurethanen, Acrylaten oder MBS modifiziert sind.
13. Polyphenylenoxide und -sulfide und deren Mischungen mit Styrolpolymeren oder Polyamiden.
14. Polyurethane, die sich von Polyethern, Polyestern und Polybutadienen mit endständigen Hydroxylgruppen einerseits und aliphatischen oder aromatischen Polyisocyanaten andererseits ableiten, sowie deren Vorprodukte.
15. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, 6/10, 6/9, 6/12, 4/6, Polyamid 11, Polyamid 12, aromatische Polyamide ausgehend von m-Xylol, Diamin und Adipinsäure; Polyamide, hergestellt aus Hexamethylendiamin und Iso- und/oder Terephthalsäure und gegebenenfalls einem Elastomer als Modifikator, z.B. Poly-2,4,4-trimethylhexamethylenterephthalamid, Poly-m-phenylen-isophthalamid. Block-Copolymere der vorstehend genanntenPolyamide mit Polyolefinen, Olefin-Copolymeren, Ionomeren oder chemisch gebundenen oder gepfropften Elastomeren; oder mit Polyethern, wie z.B. mit Polyethylenglykol, Polypropylenglykol oder Polytetramethylenglykol. Ferner mit EPDM oder ABS modifizierte Polyamide oder Copolyamide; sowie während der Verarbeitung kondensierte Polyamide ("RIM-Polyamidsysteme").
16. Polyharnstoffe, Polyimide, Polyamid-imide und Polybenzimidazole.
17. Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Polyhydroxybenzoate, sowie Block-Polyether-ester, die sich von Polyethern mit Hydroxylendgruppen ableiten; ferner mit Polycarbonaten oder MBS modifizierte Polyester.
18. Polycarbonate und Polyestercarbonate.
19. Polysulfone, Polyethersulfone und Polyetherketone.
20. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.
21. Trocknende und nicht-trocknende Alkydharze.
22. Ungesättigte Polyesterharze, die sich von Copolyestern gesättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.
23. Vernetzbare Acrylharze, die sich von substituierten Acrylsäureestern ableiten, wie z.B. von Epoxyacrylaten, Urethan-acrylaten oder Polyester-acrylaten.
24. Alkydharze, Polyesterharze und Acrylatharze, die mit Melaminharzen, Harnstoffharzen, Polyisocyanaten oder Epoxidharzen vernetzt sind.
25. Vernetzte Epoxidharze, die sich von Polyepoxiden ableiten, z.B. von Bis-glycidylethern oder von cycloaliphatischen Diepoxiden.
26. Naturliche Polymere, wie Cellulose, Naturkautschuk, Gelatine, sowie deren polymer-homolog chemisch abgewandelte Derivate, wie Celluloseacetate, -propionate und -butyrate, bzw. die Celluloseether, wie Methylcellulose; sowie Kolophoniumharze und Derivate.
27. Mischungen (Polyblends) der vorgenannten Polymeren, wie z.B. PP/EPDM, Polyamid/EPDM oder ABS, PVC/EVA, PVC/ABS, PVC/MBS, PC/ABS, PBTP/ABS, PC/ASA, PC/PBT, PVC/CPE, PVC/Acrylate, POM/thermoplastisches PUR, PC/thermoplastisches PUR, POM/Acrylat, POM/MBS, PPO/HIPS, PPO/PA 6.6 und Copolymere, PA/HDPE, PA/PP, PAF/PPO.
28. Natürliche und synthetische organische Stoffe, die reine monomere Verbindungen oder Mischungen von solchen darstellen, beispielsweise Mineralöle, tierische oder pflanzliche Fette, Oele und Wachse, oder Oele, Wachse und Fette auf Basis synthetischer Ester (z.B. Phthalate, Adipate, Phosphate oder Trimellitate), sowie Abmischungen synthetischer Ester mit Mineralölen in beliebigen Gewichtsverhältnissen, wie

sie z.B. als Spinnpräparationen Anwendung finden, sowie deren wässrige Emulsionen.

29. Wässrige Emulsionen natürlicher oder synthetischer Kautschuke, wie z.B. Naturkautschuk-Latex oder Latices von carboxylierten Styrol-Butadien-Copolymeren.

Weitere Gegenstände der Erfindung sind daher Zusammensetzungen enthaltend ein gegen oxidativen, thermischen oder aktinischen Abbau empfindliches organisches Material und mindestens eine Verbindung der Formeln Ia und/oder Ib, sowie die Verwendung von Verbindungen der Formeln Ia und Ib zum Stabilisieren von organischem Material gegen oxidativen, thermischen oder aktinischen Abbau.

Bevorzugte organische Materialien sind Polymere, z.B. synthetische Polymere, insbesondere thermoplastische Polymere. Besonders bevorzugte organische Materialien sind Polyolefine, z.B. die oben unter den Punkten 1 bis 3 angegebenen, insbesondere Polyethylen und Polypropylen.

Im allgemeinen werden die Verbindungen der Formeln Ia und Ib dem zu stabilisierenden Material in Mengen von 0,01 bis 10 %, bevorzugt 0,01 bis 5 %, insbesondere 0,01 bis 2 %, bezogen auf das Gesamtgewicht des zu stabilisierenden Materials, zugesetzt.

Neben den Verbindungen der Formeln Ia und/oder Ib können die erfindungsgemässen Zusammensetzungen zusätzlich herkömmliche Additive enthalten, wie beispielsweise die unten angegebenen.

1. Antioxidantien

1.1. Alkylierte Monophenole, z.B. 2,6-Di-tert.butyl-4-methylphenol, 2-Tert.butyl-4,6-dimethylphenol, 2,6-Di-tert.butyl-4-ethylphenol, 2,6-Di-tert.butyl-4-n-butylphenol, 2,6-Di-tertbutyl-4-i-butylphenol, 2,6-Di-cyclopentyl-4-methylphenol, 2-(α-Methylcyclohexyl)-4,6-dimethylphenol, 2,6-Di-octadecyl-4-methylphenol, 2,4,6-Tri-cyclohexylphenol, 2,6-Di-tert.butyl-4-methoxymethylphenol, 2,6-Di-nonyl-4-methylphenol.

1.2. Alkylierte Hydrochinone, z.B. 2,6-Di-tert.butyl-4-methoxyphenol, 2,5-Di-tert.butylhydrochinon, 2,5-Di-tert.butyl-hydrochinon, 2,6-Diphenyl-4-octadecyloxyphenol.

1.3. Hydroxylierte Thiodiphenylether, z.B. 2,2′-Thio-bis-(6-tert.-butyl-4-methylphenol), 2,2′-Thio-bis-(4-octylphenol), 4,4′-Thio-bis-(6-tert.butyl-3-methylphenol), 4,4′-Thio-bis(6-tert.butyl-2-methylphenol).

1.4. Alkyliden-Bisphenole, z.B. 2,2′-Methylen-bis-(6-tertbutyl-4-methylphenol), 2,2′-Methylen-bis-(6-tert.butyl-4-ethylphenol), 2,2′-Methylen-bis-[4-methyl-6-(α-methylcyclohexyl)-phenol], 2,2′-Methylen-bis-(4-methyl-6-cyclohexylphenol), 2,2′-Methylen-bis-(6-nonyl-4-methylphenol), 2,2′-Methylen-bis-(4,6-di-tert.butylphenol), 2,2′-Ethyliden-bis-(4,6-di-tert.butylphenol), 2,2′-Ethyliden-bis-(6-tert.butyl-4-isobutylphenol), 2,2′-Methylen-bis-[6-(α-methylbenzyl)-4-nonylphenol], 2,2′-Methylen-bis-[6-(α,α-dimethylbenzyl)-4-nonylphenol], 4,4′-Methylen-bis-(2,6-di-tert.-butylphenol), 4,4′-Methylen-bis-(6-tert.butyl-2-methylphenol), 1,1-Bis-(5-tert.butyl-4-hydroxy-2-methylphenyl)-butan, 2,6-Bis-(3-tert.butyl-5-methyl-2-hydroxybenzyl)-4-methylphenol, 1,1,3-Tris-(5-tert.butyl-4-hydroxy-2-methylphenyl)-butan, 1,1-Bis-(5-tert.butyl-4-hydroxy-2-methyl-phenyl)-3-n-dodecylmercaptobutan, Ethylenglycol-bis-[3,3-bis-(3′-tert-butyl-4′-hydroxyphenyl)-butyrat], Bis-(3-tert.butyl-4-hydroxy-5-methyl-phenyl)-dicyclopentadien, Bis-[2-(3′-tertbutyl-2′-hydroxy-5′-methyl-benzyl)-6-tert.butyl-4-methyl-phenyl]-terephthalat.

1.5 Benzylverbindungen, z.B. 1,3,5-Tris-(3,5-di-tert.butyl-4-hydroxy-benzyl)-2,4,6-trimethylbenzol, Bis-(3,5-di-tert.butyl-4-hydroxybenzyl)-sulfid, 3,5-Di-tert.butyl-4-hydroxybenzyl-mercaptoessigsäure-isooctylester, Bis-(4-tert.butyl-3-hydroxy-2,6-dimethylbenzyl)dithiol-terephthalat, 1,3,5-Tris-(3,5-di-tert.butyl-4-hydroxybenzyl)-isocyanurat, 1,3,5-Tris-(4-tert.butyl-3-hydroxy-2,6-dimethylbenzyl)-isocyanurat, 3,5-Di-tert.butyl-4-hydroxybenzyl-phosphonsäure-dioctadecylester, Ca-Salz des 3,5-Di-tert.butyl-4-hydroxybenzyl-phosphonsäure-monoethylester, 1,3,5-Tris-(3,5-dicyclohexyl-4-hydroxybenzyl)isocyanurat.

1.6. Acylaminophenole, z.B. 4-Hydroxy-laurinsäureanilid, 4-Hydroxystearinsäureanilid, 2,4-Bis-(octylmercapto)-6-(3,5-di-tert.butyl-4-hydroxyanilino)-s-triazin, N-(3,5-di-tert.butyl-4-hydroxyphenyl)-carbaminsäureoctylester.

1.7. Ester der β-(3,5-Di-tert.butyl-4-hydroxyphenyl)-propionsäure mit ein- oder mehrwertigen Alkoholen, wie z.B. mit Methanol, Octa-decanol, 1,6-Hexandiol, Neopentylglycol, Thiodiethylenglycol, Diethylenglycol, Triethylenglycol, Pentaerythrit, Tris-(hydroxy)ethyl-isocyanurat, N,N′-Bis-(hydroxyethyl)-oxalsäurediamid.

1.8. Ester der β-(5-tert.butyl-4-hydroxy-3-methylphenyl)-propionsäure mit ein- oder mehrwertigen Alkoholen, wie z.B. mit Methanol, Octadecanol, 1,6-Hexandiol, Neopentylglycol, Thiodiethylenglycol, Diethylenglycol, Triethylenglycol, Pentaerythrit, Tris-(hydroxy)ethyl-isocyanurat, N,N′-Bis-(hydroxyethyl)-oxalsäurediamid.

1.9. Ester der β-(3,5-Dicyclohexyl-4-hydroxyphenyl)-propionsäure mit ein- oder mehrwertigen Alkoholen, wie z.B. mit Methanol, Octadecanol, 1,6-Hexandiol, Neopentylglycol, Thiodiethylenglycol, Diethylenglycol, Triethylenglycol, Pentaerythrit, Tris-(hydroxy)ethyl-isocyanurat, N,N′-Bis-(hydroxyethyl)-oxalsäurediamid.

<u>1.10. Amide der β-(3,5-Di-tert.butyl-4-hydroxyphenyl)-propionsäure</u>, wie z.B. N,N'-Bis-(3,5-di-tert.butyl-4-hydroxyphenylpropionyl)-hexamethylendiamin, N,N'-Bis-(3,5-di-tert.-butyl-4-hydroxyphenylpropionyl)-trimethylendiamin, N,N'-Bis-(3,5-di-tert.butyl-4-hydroxyphenylpropionyl)-hydrazin.

<u>2. UV-Absorber und Lichtschutzmittel</u>

<u>2.1. 2-(2'-Hydroxyphenyl)-benztriazole</u>, wie z.B. das 5'-Methyl-, 3',5'-Di-tert.butyl-, 5'-tert.Butyl-, 5'-(1,1,3,3-Tetramethylbutyl)-, 5-Chlor-3',5'-di-tert.butyl-, 5-Chlor-3'-tert.butyl-5'-methyl-, 3'-sec.Butyl-5'-tert.butyl, 4'-Octoxy-, 3',5'-Di-tert.amyl-, 3',5'-Bis-(α,α-dimethylbenzyl)-Derivat.

<u>2.2. 2-Hydroxybenzophenone</u>, wie z.B. das 4-Hydroxy-, 4-Methoxy-, 4-Octoxy-, 4-Decyloxy-, 4-Dodecyloxy-, 4-Benzyloxy-, 4,2',4'-Trihydroxy-, 2'-Hydroxy-4,4'-dimethoxy-Derivat.

<u>2.3. Ester von gegebenenfalls substituierten Benzoesäuren</u>, wie z.B. 4-tert.Butyl-phenylsalicylat, Phenylsalicylat, Octylphenyl-salicylat, Dibenzoylresorcin, Bis-(4-tert.butylbenzoyl)-resorcin, Benzoylresorcin, 3,5-Di-tert.butyl-4-hydroxybenzoesäure-2,4-di-tert.butylphenylester, 3,5-Di-tert.butyl-4-hydroxybenzoesäurehexadecylester.

<u>2.4. Acrylate</u>, wie z.B. α-Cyan-β,β-diphenylacrylsäure-ethylester bzw. -isooctylester, α-Carbomethoxy-zimtsäuremethylester, α-Cyano-β-methyl-p-methoxy-zimtsäuremethylester bzw. -butylester, α-Carbomethoxy-p-methoxy-zimtsäure-methylester, N-(β-Carbomethoxy-β-cyanovinyl)-2-methyl-indolin.

<u>2.5. Nickelverbindungen</u>, wie z.B. Nickelkomplexe des 2,2'-Thio-bis-[4-(1,1,3,3-tetramethylbutyl)-phenols], wie der 1:1- oder der 1:2-Komplex, gegebenenfalls mit zusätzlichen Liganden, wie n-Butylamin, Triethanolamin oder N-Cyclohexyl-diethanolamin, Nickeldibutyldithiocarbamat, Nickelsalze von 4-Hydroxy-3,5-di-tert.butylbenzylphosphonsäure-monoalkylestern, wie vom Methyl- oder Ethylester, Nickelkomplexe von Ketoximen, wie von 2-Hydroxy-4-methyl-phenyl-undecylketoxim, Nickelkomplexe des 1-Phenyl-4-lauroyl-5-hydroxy-pyrazols, gegebenenfalls mit zusätzlichen Liganden.

<u>2.6. Sterisch gehinderte Amine</u>, wie z.B. Bis-(2,2,6,6-tetramethyl-piperidyl)-sebacat, Bis-(1,2,2,6,6-pentamethylpiperidyl)-sebacat, n-Butyl-3,5-di-tert.butyl-4-hydroxybenzylmalonsäure-bis(1,2, 2,6,6-pentamethylpiperidyl)-ester, Kondensationsprodukt aus 1-Hydroxyethyl-2,2,6,6-tetramethyl-4-hydroxypiperidin und Bernsteinsäure, Kondensationsprodukt aus N,N'-Bis-(2,2,6,6-Tetramethyl-4-piperidyl)-hexamethylendiantin und 4-tert.Octylamino-2,6-dichlor-1,3,5-s-triazin, Tris-(2,2,6,6-tetramethyl-4-piperidyl)-nitrilotriacetat, Tetrakis-(2,2,6,6-tetramethyl-4-piperidyl)-1,2,3,4-butantetraoat, 1,1'-(1,2-Ethandiyl)-bis-(3,3,5,5-tetramethyl-piperazinon).

<u>2.7. Oxalsäurediamide</u>, wie z.B. 4,4'-Di-octyloxy-oxanilid, 2,2'-Di-octyloxy-5,5'-di-tert.- butyl-oxanilid, 2,2'-Di-dodecyloxy-5,5'di-tert.butyl-oxanilid, 2-Ethoxy-2'-ethyl-oxanilid, N,N'-Bis-(3-dimethylaminopropyl)-oxalamid, 2-Ethoxy-5-tert.butyl-2'-ethyloxanilid und dessen Gemisch mit 2-Ethoxy-2'-ethyl-5,4'-di-tert.butyl-oxanilid, Gemische von o- und p-Methoxy- sowie von o- und p-Ethoxy-di-substituierten Oxaniliden.

<u>2.8.2-(2-Hydroxyphenyl)-1,3,5-triazine</u>, wie z.B. 2,4,6-Tris(2-hydroxy-4-octyloxyphenyl)-1,3,5-triazin, 2-(2-Hydroxy-4-octyloxyphenyl)-4,6-bis-(2,4-dimethylphenyl)-1,3,5-triazin, 2-(2,4-Dihydroxyphenyl)-4,6-bis(2,4-dimethylphenyl)- 1,3,5-triazin, 2,4-Bis-(2-hydroxy-4-propyloxyphenyl)-6-(2,4-dimethylphenyl)-1,3,5-triazin, 2-(2-Hydroxy-4-octyloxyphenyl)-4,6-bis(4-methylphenyl)-1,3,5-triazin, 2-(2-Hydroxy-4-dodecyloxyphenyl)-4,6-bis(2,4-dimethylphenyl)-1,3,5-triazin.

<u>3. Metalldesaktivatoren</u>, wie z.B. N,N'-Diphenyloxalsäurediamid, N-Salicylal-N'-salicyloylhydrazin, N,N'-Bis-(salicyloyl)-hydrazin, N,N'-Bis-(3,5-di-tert.butyl-4-hydroxyphenylpropionyl)-hydrazin, 3-Salicyloylamino-1,2,4-triazol, Bis-(benzyliden)-oxalsäuredihydrazid.

<u>4. Phosphite und Phosphonite</u>, wie z.B. Triphenylphosphit, Diphenylalkylphosphite, Phenyldialkylphosphite, Tris-(nonylphenyl)-phosphit, Trilaurylphosphit, Trioctadecylphosphit, Distearyl-pentaerythritdiphosphit, Tris-(2,4-di-tert.butylphenyl)-phosphit, Diisodecylpentaerythrit-diphosphit, Bis-(2,4-di-tert.butylphenyl)-pentaerythritdiphosphit, Tristearyl-sorbit-triphosphit, Tetrakis-(2,4-di-tert.butylphenyl)-4,4'-biphenylen-diphosphonit, 3,9-Bis-(2,4-di-tert.butylphenoxy)-2,4,8,10-tetraoxa-3,9-diphosphaspiro[5.5]undecan.

<u>5. Peroxidzerstörende Verbindungen</u>, wie z.B. Ester der β-Thio-dipropionsäure, beispielsweise der Lauryl-, Stearyl-, Myristyl- oder Tridecylester, Mercaptobenzimidazol, das Zinksalz des 2-Mercaptobenzimidazols, Zink-dibutyl-dithiocarbamat, Dioctadecyldisulfid, Pentaerythrit-tetrakis-(β-dodecylmercapto)-propionat.

<u>6. Polyamidstabilisatoren</u>, wie z.B. Kupfersalze in Kombination mit Jodiden und/oder Phosphorverbindungen und Salze des zweiwertigen Mangans.

<u>7. Basische Co-Stabilisatoren</u>, wie z.B. Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Stearat, Na-Ricinoleat, K-Palmitat, Antimonbrenzcatechinat oder Zinnbrenzcatechinat.

8. Nukleierungsmittel, wie z.B. 4-tert.Butylbenzoesäure, Adipinsäure, Diphenylessigsäure.
9. Füllstoffe und Verstärkungsmittel, wie z.B. Calciumcarbonat, Silikate, Glasfasern, Asbest, Talk, Kaolin, Glimmer, Bariumsulfat, Metalloxide und -hydroxide, Russ, Graphit.
10. Sonstige Zusätze, wie z.B. Weichmacher, Gleitmittel, Emulgatoren, Pigmente, Optische Aufheller, Flammschutzmittel, Antistatika, Treibmittel.

Die herkömmlichen Additive werden beispielsweise in Konzentrationen von 0,01 bis 10 %, bezogen auf das Gesamtgewicht des zu stabilisierenden Materials, zugesetzt.

Die Einarbeitung der Verbindungen der Formeln Ia und Ib sowie gegebenenfalls weiterer Additive in das organische Material erfolgt nach bekannten Methoden; beispielsweise kann die Einarbeitung in polymeres Material vor oder während der Formgebung oder auch durch Aufbringen der gelösten oder dispergierten Verbindungen auf das polymere Material, gegebenenfalls unter nachträglichem Verdunsten des Lösungsmittels, erfolgen. Die Verbindungen der Formeln Ia und Ib können auch in Form eines Masterbatches, der diese beispielsweise in einer Konzentration von 2,5 bis 25 Gew.-% enthält, den zu stabilisierenden Materialien zugesetzt werden.

Die Verbindungen der Formeln Ia und Ib können auch vor oder während der Polymerisation oder vor der Vernetzung zugegeben werden.

Die Verbindungen der Formeln Ia und Ib können in reiner Form oder in Wachsen, Oelen oder Polymeren verkapselt in das zu stabilisierende Material eingearbeitet werden.

Die so stabilisierten Materialien können in verschiedenster Form angewendet werden, z.B. als Folien, Fasern, Bändchen, Formmassen, Profile oder als Bindemittel für Lacke, Klebstoffe oder Kitte.

Die Verbindungen der Formeln Ia und Ib können in Analogie zu bekannten Verfahren hergestellt werden, beispielsweise durch Umsetzung eines Diglycerins mit einer Verbindung der Formel III,

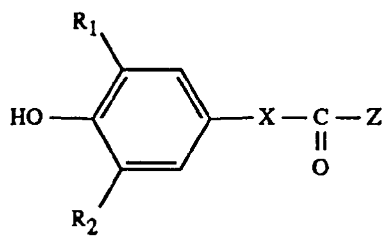

(III)

worin $R_1$, $R_2$ und X die oben angegebenen Bedeutungen besitzen und Z z.B. $C_1$-$C_4$-Alkoxy, Hydroxy oder Chlor bedeutet, in Gegenwart eines Katalysators.

Wenn Z $C_1$-$C_4$-Alkoxy bedeutet, ist es zweckmässig als Katalysatoren Dibutylzinnoxid, Titantetraisopropoxid, $LiNH_2$, LiH, LiOA, KOA oder NaOA zu verwenden, wobei A z.B. Wasserstoff, $C_1$-$C_{10}$-Alkyl oder eine gegebenenfalls durch 1 bis 3 $C_1$-$C_{10}$-Alkylreste substituierte Phenyloxygruppe darstellt Die Umsetzung wird in diesem Fall vorzugsweise in einem organischen Lösungsmittel, wie z.B. Benzol, Toluol, Xylol, Dimethylformamid u.s.w., durchgeführt. Ferner ist es vorteilhaft, die Reaktanden in einem Inertgas, z.B. Stickstoff oder Argon, vorzulegen und den bei der Umsetzung entstehenden Alkohol abzudestillieren. Zur Vervollständigung der Umsetzung kann das Reaktionsgemisch z.B. noch 8-20 Stunden bei einem Druck von 10-1013 mbar auf 180-220°C erhitzt werden.

Wenn Z Hydroxy bedeutet, wird zweckmässigerweise eine Säure, wie z.B. p-Toluolsulfonsäure, als Katalysator verwendet. Wenn Z Chlor bedeutet, ist es vorteilhaft, die Reaktion in Gegenwart eines Säureakzeptors, wie z.B. Pyridin, Triethylamin, NaOH oder KOH, durchzuführen. Die Umsetzung kann in diesem Fall z.B. bei einer Temperatur von -10° bis 150°C durchgeführt werden.

Die Verbindungen der Formel III sind bekannt und können, falls nicht im Handel erhältlich, in Analogie zu bekannten Verfahren, wie z.B. in EP-A-219 459 beschrieben, hergestellt werden.

Wird als Diglycerin ein Gemisch aus 1,2,6,7-Tetrahydroxy-4-oxaheptan und 2-Hydroxymethyl-1,5,6-trihydroxy-3-oxahexan eingesetzt, so erhält man ein Gemisch aus den Verbindungen der Formeln Ia und Ib, welches mit chromatographischen Methoden aufgetrennt werden kann.

Ein weiterer Gegenstand der Erfindung ist daher ein Gemisch aus den Verbindungen der Formeln Ia und Ib.

Ebenfalls Gegenstand der Erfindung ist ein Gemisch aus den Verbindungen der Formeln Ia und Ib, erhältlich durch Umsetzung von Diglycerin mit einem Ester der Formel III,

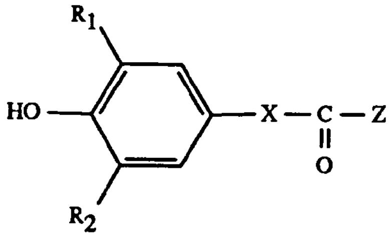

(III)

worin $R_1$, $R_2$ und X die in Anspruch 1 angegebenen Bedeutungen besitzen und Z $C_1$-$C_4$-Alkoxy, Hydroxy oder Chlor bedeutet, in Gegenwart eines Katalysators.

In dem Gemisch beträgt das Verhältnis der Verbindungen Ia/Ib z.B. 100/1 bis 1/100, bevorzugt 100/1 bis 60/10, insbesondere 90/10 bis 80/10.

Die folgenden Beispiele erläutern die Erfindung weiter. Teil- und Prozentangaben beziehen sich darin, soweit nichts anderes angegeben ist, auf das Gewicht.

Beispiel 1: Herstellung von 1,2,6,7-Tetrakis[(3′,5′-di-tert-butyl-4′-hydroxyphenyl)propionyloxy]-4-oxaheptan

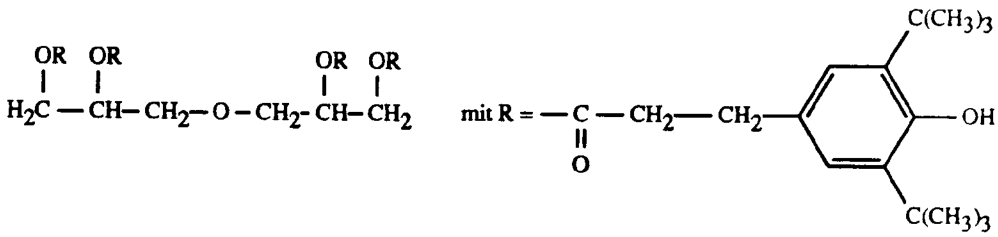

mit R =

121,6 g (0,42 Mol) Methyl-3-(3′,5′-di-tert-butyl-4′-hydroxyphenyl)propionat und 13,3 g (0,08 Mol) Diglycerin werden in einem 350 ml Sulfierkolben vorgelegt und unter Stickstoff auf 90°C erwärmt. Anschliessend werden 0,16 g Dibutylzinnoxid (= 0,8 Mol % bezogen auf das eingesetzte Diglycerin) als Katalysator zugegeben und das Reaktionsgemisch weiter auf 180°C erhitzt, wobei Methanol abdestilliert. Nach 75 Minuten wird die Apparatur langsam unter Vakuum (13,3 mbar) gesetzt und 12 Stunden auf 185°C erhitzt. Das Reaktionsgemisch wird abgekühlt und der Überschuss an eingesetztem Methyl-3-(3′,5′-di-tert-butyl-4′-hydroxyphenyl)propionat wird im Hochvakuum (2,67 x $10^{-5}$ bar) bei 123- 127°C abdestilliert. Nach säulenchromatographischer Reinigung des Rohproduktes erhält man 77,5 g (= 83 % der Theorie) eines farblosen, amorphen Pulvers mit einem Schmelzpunkt von 57°C.

Elementaranalyse:

| | | |
|---|---|---|
| Berechnet: | 73,60 % C ; | 9,18 % H |
| Gefunden: | 73,83 % C ; | 9,15 % H |

Beispiel 2: Herstellung von 1,2,6,7-Tetrakis[(3′-methyl-5′-tert-butyl-4′-hydroxyphenyl)propionyloxy]-4-oxaheptan

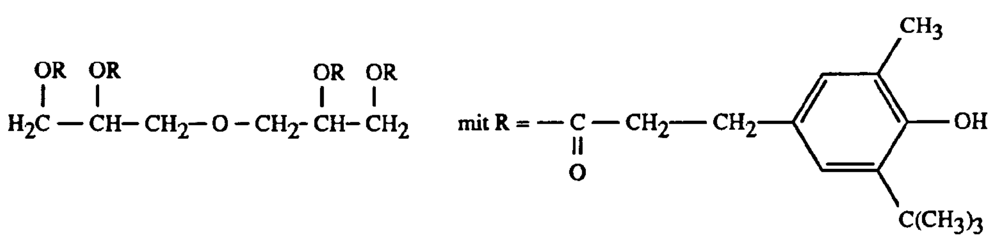

mit R =

45 g (0,18 Mol) Methyl-3-(3′-methyl-5′-tert-butyl-4′-hydroxyphenyl)propionat und 6,65 g (0,04 Mol) Diglycerin werden in einem Sulfierkolben vorgelegt und auf 100°C erhitzt. Anschliessend werden 0,1 g Dibutylzinnoxid (= 1 Mol % bezogen auf das eingesetzte Diglycerin) als Katalysator zugegeben. Das Reaktionsgemisch

wird auf 180°C erwärmt und unter Vakuum (133 mbar) 13 Stunden bei dieser Temperatur gehalten. Die Aufarbeitung erfolgt in Analogie zu der in Beispiel 1 beschriebenen Methode. Man erhält 28,2g (= 68 % der Theorie) eines amorphen Pulvers, das einen Schmelzpunkt bei 55°C besitzt.

Elementaranalyse:

| | | |
|---|---|---|
| Berechnet: | 71,65 % C ; | 8,34 % H |
| Gefunden: | 71,31 % C ; | 8,53 % H |

Beispiel 3: Stabilisierung von Polypropylen

100 Teile Polypropylenpulver, enthaltend 0,1 % Kalziumstearat, werden mit 0,3 % Dilaurylthiodipropionat und 0,1 % des in Tabelle 1 angegebenen Stabilisators gemischt und anschliessend in einem Brabender Plastographen bei 200°C 10 Minuten lang geknetet. Die so erhaltene Masse wird in einer Presse mit einer Oberflächentemperatur von 260°C zu 1 mm dicken Platten gepresst, aus denen Streifen von 1 cm Breite und 10 cm Länge gestanzt werden. Von jeder Platte werden mehrere solcher Streifen in einen auf 135°C oder 149°C geheizten Umluftofen gehängt und in regelmässigen Zeitabständen beobachtet. Die oxidative Zersetzung dieser Streifen lässt sich an einer kreisartig beginnenden Gelbfärbung erkennen. Ein Mass für die Stabilität der Probe ist die Zeitdauer in Tagen bis zur Zersetzung.

Tabelle 1:

| Verbindung aus Beispiel | Anzahl Tage Ofenalterung bis zur Zersetzung 135°C | 149°C |
|---|---|---|
| - | 1 | 1 |
| 1 | 241 | 103 |
| 2 | 189 | 90 |

**Patentansprüche**

1. Verbindungen der Formeln Ia und Ib,

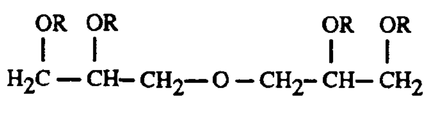 , 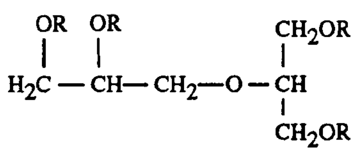

(Ia) (Ib)

worin die Reste R gleich oder verschieden sind und eine Gruppe der Formel II bedeuten,

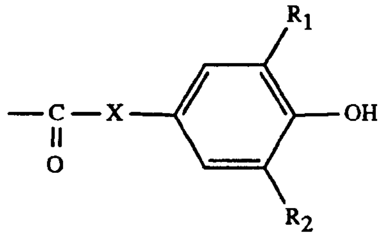

(II)

worin X eine direkte Bindung, Methylen, Ethylen oder eine Gruppe

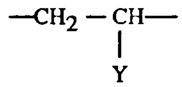

bedeutet und Y $C_1$-$C_8$-Alkyl ist,
$R_1$ und $R_2$ unabhängig voneinander $C_1$-$C_{18}$-Alkyl, $C_5$-$C_{12}$-Cycloalkyl, durch $C_1$-$C_4$-Alkyl substituiertes $C_5$-$C_7$-Cycloalkyl, Phenyl oder $C_7$-$C_{10}$-Phenylalkyl sind und $R_2$ zusätzlich Wasserstoff bedeutet.

2. Verbindungen gemäss Anspruch 1, worin die Reste R gleich sind.

3. Verbindungen gemäss Anspruch 1, worin X eine direkte Bindung, Methylen oder Ethylen ist.

4. Verbindungen gemäss Anspruch 1, worin $R_1$ und $R_2$ unabhängig voneinander $C_1$-$C_{12}$-Alkyl, $C_5$-$C_7$-Cycloalkyl, durch $C_1$-$C_4$-Alkyl substituiertes $C_5$-$C_7$-Cycloalkyl, Phenyl, Benzyl oder Phenylethyl bedeuten und $R_2$ zusätzlich Wasserstoff ist.

5. Verbindungen gemäss Anspruch 1, worin X eine direkte Bindung, Methylen oder Ethylen ist, $R_1$ und $R_2$ unabhängig voneinander $C_1$-$C_4$-Alkyl, Cyclohexyl, durch $C_1$-$C_4$-Alkyl substituiertes Cyclohexyl, Phenyl oder Benzyl bedeuten und $R_2$ zusätzlich Wasserstoff ist.

6. Verbindungen gemäss Anspruch 1, worin X Ethylen bedeutet, $R_1$ und $R_2$ unabhängig voneinander $C_1$-$C_4$-Alkyl sind und $R_2$ zusätzlich Wasserstoff bedeutet.

7. Verbindungen gemäss Anspruch 1, worin die Reste R gleich sind und 3,5-Di-tert-butyl-4-hydroxyphenylpropionyloxy oder 3-methyl-5-tert-butyl-4-hydroxyphenylpropionyloxy bedeuten.

8. Ein Gemisch aus den in Anspruch 1 definierten Verbindungen der Formeln Ia und Ib.

9. Ein Gemisch aus den Verbindungen der Formeln Ia und Ib gemäss Anspruch 1, erhältlich durch Umsetzung von Diglycerin mit einem Ester der Formel III,

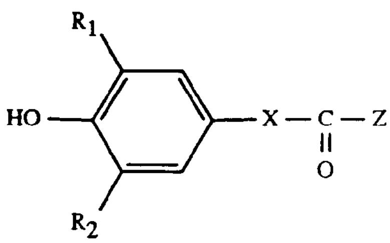

(III)

worin $R_1$, $R_2$ und X die in Anspruch 1 angegebenen Bedeutungen besitzen und Z $C_1$-$C_4$-Alkoxy, Hydroxy oder Chlor bedeutet, in Gegenwart eines Katalysators.

10. Zusammensetzung enthaltend ein gegen thermischen, oxidativen oder aktinischen Abbau empfindliches organisches Material und mindestens eine Verbindung der Formel Ia und/oder Ib gemäss Anspruch 1.

11. Zusammensetzung gemäss Anspruch 10, worin das organische Material ein Polyolefin ist.

12. Verwendung von Verbindungen der Formeln Ia und Ib gemäss Anspruch 1 zum Stabilisieren von organischem Material gegen thermischen, oxidativen oder aktinischen Abbau.

## Claims

1. A compound of the formula Ia or Ib

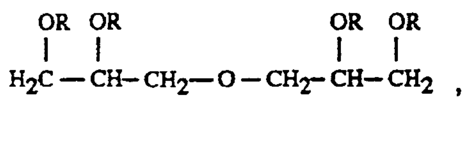

(Ia)

,

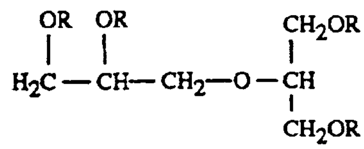

(Ib)

in which the radicals R are identical or different and are a group of the formula II

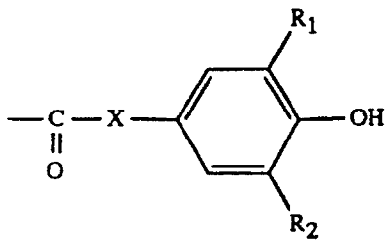

(II)

in which X is a direct bond, methylene, ethylene or a group

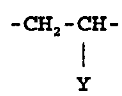

and Y is $C_1$-$C_8$alkyl,
$R_1$ and $R_2$ independently of one another are $C_1$-$C_{18}$alkyl, $C_5$-$C_{12}$cycloalkyl, $C_5$-$C_7$cycloalkyl which is substituted by $C_1$-$C_4$alkyl, or are phenyl or $C_7$-$C_{10}$phenylalkyl and $R_2$ is additionally hydrogen.

2. A compound according to claim 1, in which the radicals R are identical.

3. A compound according to claim 1, in which X is a direct bond, methylene or ethylene.

4. A compound according to claim 1, in which $R_1$ and $R_2$ independently of one another are $C_1$-$C_{12}$alkyl, $C_5$-$C_7$cycloalkyl, $C_5$-$C_7$cycloalkyl which is substituted by $C_1$-$C_4$alkyl, or are phenyl, benzyl or phenylethyl and $R_2$ is additionally hydrogen.

5. A compound according to claim 1, in which X is a direct bond, methylene or ethylene, $R_1$ and $R_2$ independently of one another are $C_1$-$C_4$alkyl, cyclohexyl, cyclohexyl which is substituted by $C_1$-$C_4$alkyl, or are phenyl or benzyl and $R_2$ is additionally hydrogen.

6. A compound according to claim 1, in which X is ethylene, $R_1$ and $R_2$ independently of one another are $C_1$-$C_4$alkyl and $R_2$ is additionally hydrogen.

7. A compound according to claim 1, in which the radicals R are identical and are 3,5-di-tert-butyl-4-hydroxyphenylpropionyloxy or 3-methyl-5-tert-butyl-4-hydroxyphenylpropionyloxy.

8. A mixture of compounds of the formulae Ia and Ib defined in claim 1.

9. A mixture of compounds of the formulae Ia and Ib according to claim 1, obtainable by reacting diglycerol with an ester of the formula III

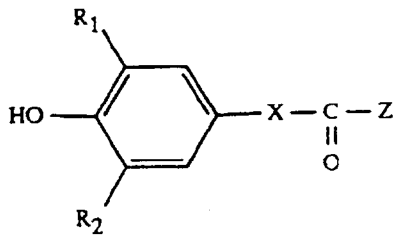

(III)

in which $R_1$, $R_2$ and X are as defined in claim 1 and Z is $C_1$-$C_4$alkoxy, hydroxyl or chlorine, in the presence of a catalyst.

**10.** A composition containing an organic material which is sensitive to thermal, oxidative or actinic degradation, and at least one compound of the formula Ia and/or Ib according to claim 1.

**11.** A composition according to claim 10, in which the organic material is a polyolefin.

**12.** The use of compounds of the formulae Ia and Ib according to claim 1 for stabilizing organic material against thermal, oxidative or actinic degradation.

## Revendications

**1.** Composés de formules Ia et Ib ci-dessous

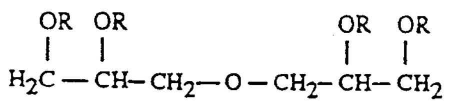 , 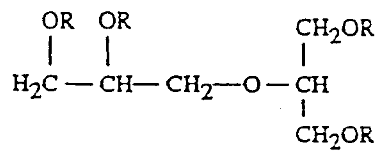

(Ia) (Ib)

dans lesquelles les radicaux R, qui peuvent être identiques ou différents les uns des autres, sont chacun un groupe de formule II

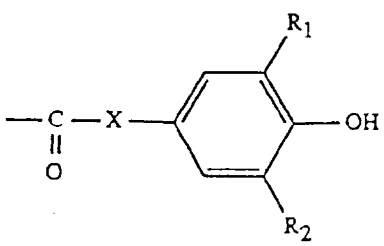

(II)

X représentant une liaison directe, un groupe méthylène ou éthylène ou un groupe
dont Y est un alkyle en $C_1$-$C_8$, et
$R_1$ et $R_2$ étant, indépendamment l'un de l'autre, un alkyle en $C_1$-$C_{18}$, un cycloalkyle en $C_5$-$C_{12}$, un cycloalkyle en $C_5$-$C_7$ avec comme substituant en alkyle en $C_1$-$C_4$, un phényle ou un phénylalkyle en $C_7$-$C_{10}$, $R_2$ pouvant être en outre l'hydrogène.

**2.** Composés selon la revendication 1 dont les radicaux R sont identiques.

**3.** Composés selon la revendication 1 dans lesquels X est une liaison directe ou le groupe méthylène ou éthylène.

**4.** Composés selon la revendication 1 dans lesquels $R_1$ et $R_2$ sont, indépendamment l'un de l'autre, un alkyle en $C_1$-$C_{12}$, un cycloalkyle en $C_5$-$C_7$, un cycloalkyle en $C_5$-$C_7$ avec comme substituant un alkyle en $C_1$-$C_4$, un phényle, un benzyle ou un phényléthyle, $R_2$ pouvant être en outre l'hydrogène.

5. Composé selon la revendication 1 dans lesquels X est une liaison directe ou le groupe méthylène et éthylène, et $R_1$ et $R_2$ sont, indépendamment l'un de l'autre, un alkyle en $C_1$-$C_4$, un cyclohexyle, un cyclohexyle avec comme substituant un alkyle en $C_1$-$C_4$, un phényle ou un benzyle, $R_2$ pouvant être en outre l'hydrogène.

6. Composés selon la revendication 1 dans lesquels X est le groupe éthylène et $R_1$ et $R_2$, indépendamment l'un de l'autre, sont chacun un alkyle en $C_1$-$C_4$, $R_2$ pouvant être en outre l'hydrogène.

7. Composés selon la revendication 1 dans lesquels les radicaux R sont identiques et sont le radical 3,5-di-tert-butyl-4-hydroxyphénylpropionyloxy ou 3-méthyl-5-tert-butyl-4-hydroxyphényl-propionyloxy.

8. Un mélange de composés de formules Ia et Ib tels que définis à la revendication 1.

9. Un mélange de composés de formules Ia et Ib selon la revendication 1, que l'on peut obtenir par réaction en présence d'un catalyseur du diglycérol avec un ester de formule III,

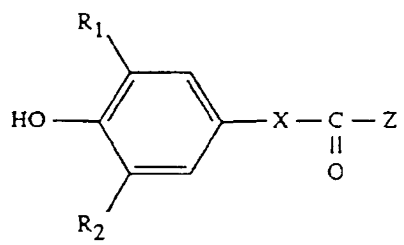

(III)

dans laquelle $R_1$, $R_2$ et X ont les significations indiquées à la revendication 1 et Z représente un groupe alcoxy en $C_1$-$C_4$, le groupe hydroxy ou un atome de chlore.

10. Composition comprenant une matière organique sensible aux dégradations par oxydation ou sous l'action de la chaleur ou d'un rayonnement actinique, avec un ou plusieurs composés de formule Ia et/ou Ib selon la revendication 1.

11. Composition selon la revendication 10 dont la matière organique est une polyoléfine.

12. L'emploi des composés de formules Ia et Ib selon la revendication 1 pour protéger des matières organiques contre les dégradations provoquées par une oxydation ou par l'action de la chaleur ou d'un rayonnement actinique.